Europäisches Patentamt

European Patent Office

Office européen des brevets

(19)

(11) Publication number: **0 335 844**
**A2**

(12) # EUROPEAN PATENT APPLICATION

(21) Application number: **89850001.2**

(22) Date of filing: **02.01.89**

(51) Int. Cl.⁴: **A 61 K 31/08**

(30) Priority: **07.01.88 SE 8800026**

(43) Date of publication of application:
**04.10.89 Bulletin 89/40**

(84) Designated Contracting States:
**AT BE CH DE ES FR GB GR IT LI LU NL SE**

(71) Applicant: **HÄLSOPRODUKTER LARS KÄRNERUD AB**
**Box 145**
**S-57022 Forserum (SE)**

(72) Inventor: **Brohult, Sven**
**Gliavägen 97**
**S-161 52 Bromma (SE)**

**Brohult, Astrid**
**Gliavägen 97**
**S-161 52 Bromma (SE)**

(74) Representative: **Arwidi, Bengt**
**AHLPATENT AB Hemstigen 21**
**S-552 66 Jönköping (SE)**

The title of the invention has been amended (Guidelines for Examination in the EPO, A-III, 7.3).

(54) **Use of glycerol ethers in the treatment of AIDS.**

(57) This invention relates to use of at least one glycerol ether for a pharmaceutical preparation for treatment of AIDS and HIV-infected persons. The pharmaceutical is administered orally using capsulus. The active substance comprises a mixture of glycerol ethers and is produced from shark liver.

EP 0 335 844 A2

## Description

### Use of glycerol ether

This invention relates to use of at least one glycerol ether for a pharmaceutical preparation. The pharmaceutical preparation is for treatment of AIDS and HIV-infected persons.

Glycerol ethers (previously also called alcoxyglycerols) have shown several important medical effects. Oral administration of glycerol ethers by radiation therapy reduces the number of harmful radiation effects; leucopenia and trombocytopenia is partly or fully prevented. By certain tumor diseases a lower mortality is obtained in a group which has been given glycerol ethers as profylactic treatment as compared to a group which was not given these substances. Experiments both with humans and animals have shown that the glycerol ethers improve the bodily immunity defense mechanism.

In a human body glycerol ethers are found mainly in bone marrow, liver, mother's milk and placenta. The glycerol ethers are found in larger quantities mainly in shark liver oil.

The glycerol ethers have the following general formula:

$CH_2 . OH$

$CH . OH$

$CH_2 . O . R,$

wherein R represents a longchained, aliphatic hydrocarbon chain. Naturally occurring glycerol ethers are mixtures with varying number of carbon atoms in the side chain. Compounds having 16 or 18 carbon atoms make up most the quantities, but the number of carbon atoms varies mainly with the interval of 14 - 24. E.g. chimmyl alcohol and batyl alcohol are saturated glycerol ethers having 16 and 18 carbon atoms respectively in the side chain. Selachyl alcohol is an unsaturated glycerol et with 18 carbon atoms. There also are found methoxysubstituted glycerol ethers, i.e. compounds wherein one hydrogen atom, mostly carbon atom 2, has been replaced by a methoxy group ($-OCH_3$).

In the human body like in shark liver oil the glycerol ethers are present as di-esters of fatty acids. They differ from ordinary fat (triglycerid, i.e. a tri-ester of fatty acids) onlyat one point in the molecule, namely that one of the three ester bonds is replaced by an ether bond. HIV (human immunity deficiency virus) causes - often after several years - the AIDS disease. HIV-infected persons and those who have already developed AIDS often show a rapid weight loss - 10 to 15 kg.

It has now unexpected been found that this weight loss stops by the administration of glycerol ethers. After 3 to 4 months the infected persons have almost regained normal weight.

The glycerol ethers have been administered orally using capsules, which are available under the trademark ECOMER. Each capsule contains 0.05 g of the active substance, which comprises a mixture of glycerol ethers and is produced from shark liver.

## Claims

1) Use of at least one glycerol ether for making a pharmaceutical preparation for treatment of AIDS and HIV-infected persons.

2) Use according to claim 1, **characterized** in that the mixture of glycerol ethers being present in shark liver oil is used.

3) Use according to claim 1, **characterized** in that one or more glycerol ethers with a straight side chain is used.

4) Use according to claim 1, **characterized** in that one or more methoxy-substituted glycerol ethers is used.

5) Use according to claim 1, **characterized** in that a mixture of glycerol ethers having straight side chains and methoxy-substituted glycerol ethers is used.